# FASCICULE DE BREVET EUROPEEN

(11) **EP 1 140 000 B1**
(45) Date de publication et mention de la délivrance du brevet: **26.10.2005**
(21) Numéro de dépôt: 99964734.0
(22) Date de dépôt: 29.12.1999
(51) Int. Cl.: A61K 7/42, A61K 7/48

(54) **COMPOSITION COSMETIQUE OU DERMATOLOGIQUE CONTENANT UN ACTIF STIMULANT LA SYNTHESE DE LA PROTEINE HSP 32 DANS LA PEAU ET METHODE DE TRAITEMENT COSMETIQUE**
KOSMETISCHE UND DERMATOLOGISCHE ZUSAMMENSETZUNG ENTHALTEND EINEN WIRKSTOFF ZUR STIMULIERUNG DER SYNTHESE DES HSP 32 PROTEINS IN DER HAUT UND VERFAHREN ZUR KOSMETISCHEN BEHANDLUNG
COSMETIC OR DERMATOLOGICAL COMPOSITION CONTAINING AN ACTIVE PRINCIPLE STIMULATING HSP 32 PROTEIN SYNTHESIS IN THE SKIN AND COSMETIC TREATMENT METHOD

(30) Priorité: 30.12.1998 FR 9816641
(43) Date de publication de la demande: 10.10.2001
(73) Titulaire: PARFUMS CHRISTIAN DIOR, 75008 Paris (FR)
(72) Inventeur: NIZARD, Carine, F-94200 Ivry S/Seine (FR); MOREAU, Marielle, F-78770 Marcq (FR); BONTE, Frédéric, F-45100 Orléans (FR)
(74) Mandataire: Warcoin, Jacques
(86) Numéro de dépôt international: PCT/FR1999/003310
(87) Numéro de publication internationale: WO 2000/040215

(56) Documents cités:
- WO-A-92/16544
- FR-A- 2 652 086
- FR-A- 2 687 572
- FR-A- 2 699 818
- FR-A- 2 708 851
- FR-A- 2 757 863

## Description

La présente invention se rapporte à des compositions, notamment dermato-cosmétologiques, utiles dans le domaine de la photoprotection et à des méthodes de traitement cosmétique de la peau exposée au rayonnement solaire.

Les radiations solaires, et principalement les rayonnements ultraviolets peuvent entraîner des phénomènes néfastes, à moyen ou long terme. L'énergie solaire atteignant le sol se répartit, à des longueurs d'onde (λ) de 290 à 2500 nm, pour 50% dans l'infra-rouge (λ = 800 à 2500 nm), pour 40% dans le visible ((λ = 400 à 800 nm) et pour 10% dans l'ultraviolet, où l'on distingue les UVA ((λ = 320 - 400 nm) et les UVB ((λ = 290 - 320 nm).

Si la pigmentation, immédiate (UVA) ou retardée (UVB), constitue un moyen de défense naturelle de la peau, l'exposition aux rayonnements ultraviolets peut entraîner un érythème actinique, une hyperplasie épidermique, une senéscence cutanée (ou élastose solaire) et même, dans certains cas, favoriser l'apparition de cancers cutanés.

Si la majorité du rayonnement UVB est absorbée par la couche cornée, 10% atteignent le derme ; la majorité des UVA (et du rayonnement visible) traverse l'épiderme, et 20 à 30% atteint le derme, où ils peuvent provoquer des altérations des cellules cutanées.

Il est admis que les UVA entraînent la production d'espèces d'oxygène réactif, en particulier via la génération intracellulaire d'H₂O₂ (Morlière et al., 1992).

Les cibles cellulaires de ces composés sont nombreuses et les dégâts provoqués divers :
- ADN : coupures simple ou double brin, des pontages ADN-protéines,
- Protéines : l'oxygène singulet réagit avec certains résidus dont l'histidine et le tryptophane,
- Membranes : péroxydation des acides gras polyéthyléniques.

Ces altérations ont lieu sur tous les types cellulaires cutanés, en particulier kératinocytes, mélanocytes et fibroblastes, et généralement sont des manifestations inflammatoires ou des manifestations du vieillissement actinique (rides).

Il est également reconnu que des protéines dites "HSP" (de l'anglais : "heat shock proteins") ont été mises en évidence sur des cellules, aussi bien eucaryotes que procaryotes, soumises à des stress physiologiques, en particulier thermiques, in vivo aussi bien qu'in vitro ; Ces cellules réagissent en exprimant un ensemble de protéines dont le nombre et la taille varient selon l'organisme cible et le stress inducteur (Maytin, 1995 ; Milarski et al., 1989).

Les HSP sont classées en famille selon leur poids moléculaire. On distingue alors des HSP 90, HSP 70, HSP 60, HSP 30. Plusieurs des gènes codant pour les HSP ont été séquencés et leur localisation chromosomique déterminée ; cependant peu d'informations sont actuellement disponibles concernant le contrôle transcriptionnel de ces molécules qu'on soupçonne de faire partie des dispositifs cellulaires de protection contre un environnement toxique.

De nombreux facteurs peuvent provoquer l'induction d'HSP : températures élevées, métaux lourds, infections virales, alcool, facteurs de croissance et basses températures (Simon et al., 1995). Ces différents facteurs étant tous stressants pour la cellule, les HSP sont à présent le plus souvent appelées " protéines de stress" (Maytin, 1995). Les rayons UV sont également à l'origine de l'induction de certaines HSP.

A ce jour, il est classiquement admis que la protection par voie topique de la peau vis-à-vis des rayons ultraviolets est liée à l'emploi de filtres physiques ou chimiques dans les produits solaires ou destinés à prévenir le vieillissement.

Or, la présente invention repose sur une approche nouvelle, complémentaire de celle de la protection par les filtres anti-ultraviolets. Il s'agit, en effet, de compléter la protection par filtres anti-ultraviolets au moyen d'une protection par le biais de l'induction de la synthèse endogène de la protéine de choc thermique HSP 32. Plus particulièrement la présente invention concerne des compositions dermatologiques ou cosmétologiques, caractérisées en ce qu'elles contiennent au moins un composé susceptible d'activer la synthèse endogène de HSP 32 ou d'un fragment peptidique fonctionnel d'une telle protéine, avec des excipients pharmaceutiquement et/ou cosmétologiquement acceptables, un filtre UVA ou UVB et la forskoline ou un extrait en contenant.

Par "composé susceptible d'activer la synthèse endogène de HSP 32 ou d'un fragment peptidique fonctionnel d'une telle protéine", on entend essentiellement désigner tous les composés susceptibles de favoriser la production endogène de HSP 32 et particulièrement les molécules impliquées dans le rétrocontrôle positif de la synthèse ainsi que les précurseurs de la matière active ou encore les oligodésoxynucléotides ou oligoribonucléotides.

Les compositions selon l'invention contiennent au moins un filtre des rayons ultraviolets A et/ou ultraviolets B. Ces filtres sont bien connus de l'homme de l'art. Citons, à titre d'exemple, les benzophénones, telles que la 2,2',4,4'-tetrahydroxy-benzophénone ou Benzophénone-2 et la 2-hydroxy-4'-méthoxy-benzophénone ou Eusolex 4360 ®, qui absorbe les UVA et les UVB, les dérivés cinnamates tels que l'octyl-p-méthoxycinnamate ou Parsol MCX ®, qui absorbe les UVB, les dérivés dibenzoylméthane tels que le 4-tert-butyl-4'-méthoxydibenzoylméthane ou Parsol 1789 ®, qui absorbe les ultraviolets A. et les esters d'acides para-aminobenzoïque (Paba), tels que l'octyldiméthyl-PABA ou Escalol 507 ®, qui absorbe les UVB.

A cet égard, il est important de noter que les fibroblastes, cellules majeures du derme conférant à la peau sa tonicité, sont les seules cellules cutanées dans lesquelles il est particulièrement intéressant d'induire la production de la protéine HSP 32. Il est ainsi particulièrement intéressant pour restaurer ou conserver un bon état physiologique de la peau de stimuler la formation de cette protéine par les fibroblastes.

C'est pourquoi la présente invention concerne également l'utilisation d'un composé susceptible d'activer la synthèse endogène de HSP 32 pour la préparation d'une composition cosmétique pour la protection des fibroblastes. Parmi les composés décrits dans l'art antérieur susceptibles d'activer la synthèse des HSP en général, il convient de citer la demande de brevet FR 2 757 863 qui décrit l'utilisation de substances biologiques d'origine végétale extraites de fruits de plantes à métabolisme CAM.

Parmi les composés susceptibles de favoriser la production endogène d'HSP 32 par les fibroblastes, on peut citer les esters de l'acide caféïque et leurs dérivés, en particulier l'oraposide qui a été décrit dans les documents WO 92/16544, FR 2 652 086, FR 2 708 851, FR 2 699 818 ainsi que les OPC (oligomères procyanidoliques) qui ont été décrits dans les documents EP 953 353, EP 955 051, EP 397 914 et l'article de J. MASQUELIER (1990, Parfums, cosmétiques, arômes N° 95, pp. 89-97) et que l'on peut extraire du raisin et du thé vert par exemple, ainsi que leurs dérivés.

Parmi les dérivés d'OPC utilisables, il faut citer également les OPC réticulés tels que décrits dans le brevet US 5 780 060.

Les composés selon la présente invention seront utilisés de préférence à des concentrations comprises entre 0,1 et 5% en poids de la composition et, de façon préférée, à des concentrations comprises entre 0,2 et 1% en poids.

Les compositions selon la présente invention pourront comporter des associations de plusieurs composés "activateurs" mais également des associations avec d'autres composants intéressants. La composition contient de la forskoline ou tout extrait en contenant, notamment les extraits de Plecthantrus barbatus.

Parmi les associations préférées, il faut citer plus particulièrement celles qui contiennent au moins un composé choisi parmi :
- la tyrosine et ses dérivés, en particulier la malyltyrosine,
- l'acide ellagique et ses dérivés ou tout extrait en contenant,
- les extraits de Centella asiatica, de Potentilla erecta et d'Eriobotrya japonica,
- les saponines de soja et les saponines de luzerne telles que les soyasapogénols,
- les isoflavones, en particulier la formononétine, la daïdzéine et la génistéine ou leur mélange,
- la vitamine C et ses dérivés, en particulier le phosphate de magnésium et de vitamine C, le tocophérol et ses esters, en particulier le gentisate de tocophérol et le phosphate de tocophérol,
- l'acide 18-β-glycyrrhétinique,
- les extraits d'Azadiracta indica,
- les curcuminoïdes, en particulier une curcumine.
   Il est intéressant de remarquer que les compositions selon la présente invention peuvent également contenir des protéines de choc thermique, notamment la protéine HSP 32 elle-même ou l'un de ses fragments actifs.
   De manière préférée, les compositions selon la présente invention se présenteront sous une forme adaptée à l'administration par voie topique cutanée.
   Ces compositions pourront notamment être sous forme de solutions, suspensions, lotions, laits, gels, crèmes, émulsions H/E, E/H ou émulsions multiples, sticks ou encore poudres, adaptés à une application sur la peau, les lèvres et/ou sur les cheveux.
   Elles comprennent les excipients nécessaires à cette formulation, tels que solvants, diluants, épaississants, tensicactifs ioniques ou non ioniques, notamment des sucro-esters, conservateurs, anti-oxydants, colorants, parfums ou, dans le cas où ils sont conditionnés eu aérosol, gaz propulseurs.
   Les compositions peuvent en outre contenir des agents adoucissants, hydratants, anti-inflammatoires, des anti-rides, notamment favorisant la synthèse du glycosaminoglycanne (GAG) ou des activateurs de bronzage.
   Avantageusement, les compositions selon l'invention contiennent un agent piégeur de radicaux libres, par exemple l'α-tocophérol ou ses esters.
   Selon l'un des modes de mise en oeuvre de l'invention, la composition contient en outre au moins un autre agent photoprotecteur, de préférence choisi dans le groupe constitué des écrans solaires et des filtres solaires.
   Les filtres sont des molécules capables d'absorber les radiations, dans une zone plus ou moins étendue du spectre solaire. Ils peuvent appartenir à différentes classes ; on peut citer de manière non limitative l'acide para-aminobenzoïque et ses dérivés, les esters de l'acide cinnamique, les dérivés de l'acide salicylique ou du benzylidène-camphre, les bentimidazoles et les dérivés du benzophénone.
   Les agents écrans utilisables sont notamment des oxydes de titane, de zinc, les dérivés de mica et le talc.
   La présence d'écrans ou de filtres dans la composition permettra d'améliorer la protection contre les rayonnements solaires, de la surface corporelle sur laquelle elle est appliquée.
   L'invention a également pour objet l'utilisation d'au moins un composé choisi dans le groupe constitué par les OPC et leurs dérivés, les esters de l'acide caféique et leurs dérivés et les mélanges de ces composés, pour la préparation d'une composition destinée à activer la synthèse endogène de HSP 32 ou d'un fragment peptidique fonctionnel d'une telle protéine.
   Les aspects préférentiels énoncés ci-dessus pour la composition en tant que telle valent également pour la composition préparée et destinée à activer la synthèse endogène de HSP 32 ou d'un fragment peptidique fonctionnel d'une telle protéine selon cette utilisation. En particulier, l'utilisation selon l'invention est caractérisée en ce que la composition contient des excipients pharmaceutiquement et/ou cosmétologiquement acceptables.
   Un autre objet de l'invention est une méthode de traitement cosmétique de la peau et des phanères en vue de les protéger des effets néfastes des rayonnements, en particulier des rayonnements ultraviolets, caractérisée en ce qu'on applique localement, avant ou au moment d'une exposition aux rayonnements, notamment les rayonnements ultraviolets, par exemple les radiations solaires, une quantité efficace d'une composition telle que décrite précédemment.
   Plus particulièrement, la méthode précédente est destinée à lutter contre la formation d'érythèmes solaires, d'allergies solaires ou de l'élastose solaire et à prévenir ou retarder l'apparition des rides dues aux effets néfastes des radiations ultraviolettes.
   Enfin, selon un autre de ses aspects, l'invention comprend l'utilisation de ces compositions à titre de médicament, notamment en dermatologie.
   Dans les exemples qui suivent, on démontrera l'effet protecteur des OPC par induction d'HSP 32 en fonction d'une administration d'UVA ou non.

### EXEMPLE 1

Les exemples suivants ont été réalisés à partir de cultures cellulaires de fibroblaste qui sont ou non soumises au traitement avec les OPC puis sur lesquelles, après irradiation avec des UVA, on dose l'induction d'HSP 32 par des moyens connus de l'homme de métier.

Ces moyens comprennent en particulier l'utilisation d'un anticorps primaire anti-HSP 32, disponible dans le commerce auprès de la société TEBU, dans une technique dite d'immunodétection.

Les résultats obtenus sont rassemblés dans le tableau ci-après.

| Influence de l'OPC sur l'expression de la protéine HSP 32 avec ou sans U VA (Western blot) | | | | | | |
|---|---|---|---|---|---|---|
| | TEMOIN | | OPC 25 µg/ml | | OPC 50 µg/ml | |
| | UV- | UV+ | UV - | UV + | UV - | UV + |
| Densité volumique | 95832 | 125208 125208 | 140935 140935 | 123165 123165 | 163328 163328 | 195552 195552 |
| Effet/témoin UV - | 100% | 131% | 147% | 128% | 170% | 204% |

On constate que les UVA induisent naturellement la synthèse de HSP 32 (protéine quantifiée par Western Blot) mais cette synthèse reste modérée. L'ajout d'OPC stimule l'induction des molécules d'HSP 32 de manière plus forte que les UVA seuls, en particulier lorsque les OPC sont utilisés à 50 µg/ml.

Le traitement des cellules avec les OPC suivi d'une irradiation UVA conduit à une stimulation massive de la production d'HSP puisqu'elle peut atteindre 204% lorsque les OPC sont utilisés à 50 µg/ml.

L'effet protecteur de ces OPC est donc clairement démontré, tant avec que sans irradiation. Ainsi, les compositions pourront être utilisées à titre préventif et/ou curatif, de préférence en combinaison avec des filtres anti-UVA et/ou anti-UVB.

### EXEMPLE 2

### Compositions cosmétiques

### Crème solaire visage bronzante et protectrice

- OPC de thé vert 0,5
- Extrait de Plecthantrus barbatus 0,05
- Tyrosine 1
- Acide hyaluronique 0,2
- Eusolex 4360 ® 8
- Glycérine 3
- Acétate d'alphatocophérol 0,2
- Excipient parfumé, qsp 100

### Crème de soin anti-rides

- Extrait de Plecthantrus barbatus 0,01
- Arginine 0,2
- Acide ellagique 0,2
- OPC de thé vert 0,4
- Extrait de Centella asiatica 0,5
- Méthoxycinnamate d'octyle 2
- Excipient, qsp 100

### BIBLIOGRAPHIE

Maytin, E. D. (1995). J. Invest. Dermato. **104**, 448-454.
Milarski, K.L., Welch, W.J., and Morimoto, R.I. (1989). J. Cell. Biol. **108**, 413-424.
Morliere, P., Moysan, A., Gaboriau, F., Santus, R., Mazière, J.C., and Dubertret, L. (1992). Path. Biol. **40**, 160-168.
Simon, M. M., Reikerstorfer, A., Schwarz, A., Krone, C., Luger, T., Jäättelä, A., and Scharz, T. (1995). J. Clin. Invest. **95**, 926-933.

## Revendications

1. Composition dermatologique ou cosmétologique pour une administration par voie topique externe, **caractérisée en ce qu'**elle contient avec des excipients pharmaceutiquement et/ou cosmétiquement acceptables :
- au moins un filtre anti-ultraviolet A et/ou anti-ultraviolet B,
- au moins un composé susceptible d'activer la synthèse endogène de HSP 32 ou d'un fragment peptidique fonctionnel d'une telle protéine, et
- la forskoline ou tout extrait en contenant, notamment les extraits de Plectranthus barbatus.

2. Composition selon la revendication 1, **caractérisée en ce que** le composé susceptible d'activer la synthèse endogène des HSP est choisi parmi les OPC et leurs dérivés, les esters de l'acide caféique et leurs dérivés.

3. Composition selon la revendication 2, **caractérisée en ce que** le dérivé d'OPC est un OPC réticulé.

4. Composition selon la revendication 2, **caractérisée en ce que** l'OPC est un OPC de pépin de raisin ou un OPC de thé vert.

5. Composition selon l'une quelconque des revendications 1 à 4, **caractérisée en ce que** ledit composé est présent à une concentration comprise entre 0,1 % et 5 % p/p dans la composition.

6. Composition selon la revendication 5, **caractérisée en ce que** ledit composé est présent à une concentration comprise entre 0,2 % et 1 % p/p dans la composition.

7. Composition selon l'une quelconque des revendications 1 à 6, **caractérisée en ce qu'**elle contient en outre au moins un autre agent photoprotecteur.

8. Composition selon l'une quelconque des revendications 1 à 7, **caractérisée en ce qu'**elle contient au moins un composé choisi dans le groupe constitué des écrans solaires, des filtres solaires et des piégeurs de radicaux libres.

9. Composition selon l'une des revendications 1 à 8, **caractérisée en ce qu'**elle contient en outre la protéine HSP 32 ou l'une de ses fragments actifs.

10. Méthode de traitement cosmétique de la peau ou des phanères, en vue de les protéger des effets néfastes des rayonnements, en particulier des rayonnements ultraviolets, **caractérisés en ce qu'**on applique localement, avant ou au moment d'une exposition auxdits rayonnements, une quantité efficace d'au moins une composition selon l'une quelconque des revendications 1 à 9.

11. Méthode selon la revendication 10, **caractérisée en ce qu'**elle est destinée à lutter contre la formation d'érythèmes solaires, d'allergies solaires ou de l'élastose solaire.

12. Méthode selon la revendication 10, **caractérisée en ce qu'**elle est destinée à prévenir ou retarder le vieillissement actinique de la peau, en particulier à prévenir ou retarder l'apparition des rides dues aux effets néfastes des radiations ultraviolettes.

## Patentansprüche

1. Dermatologische oder kosmetologische Zusammensetzung für eine Verabreichung auf topischem äußerlichem Wege, **dadurch gekennzeichnet, dass** sie mit pharmazeutisch und/oder kosmetisch annehmbaren Trägersubstanzen enthält:
- wenigstens einen Anti-Ultraviolett A- und/oder Anti-Ultraviolett-B-Filter,
- wenigstens eine Verbindung, die in der Lage ist, die endogene Synthese von HSP 32 oder eines funktionsfähigen Peptidfragments eines solchen Proteins zu aktivieren, und
- Forskolin oder einen jeglichen Extrakt, der dieses enthält, insbesondere die Extrakte von Plectranthus barbatus.

2. Zusammensetzung nach Anspruch 1, **dadurch gekennzeichnet, dass** die Verbindung, die in der Lage ist, die endogene Synthese der HSP zu aktivieren, aus den PCO (procyanidolische Oligomere) und deren Derivaten, den Kaffeesäureestern und deren Derivaten ausgewählt wird.

3. Zusammensetzung nach Anspruch 2, **dadurch gekennzeichnet, dass** das PCO-Derivat ein vernetztes PCO ist.

4. Zusammensetzung nach Anspruch 2, **dadurch gekennzeichnet, dass** das PCO ein PCO aus Traubenkernen oder ein POC aus grünem Tee ist.

5. Zusammensetzung nach einem der Ansprüche 1 bis 4, **dadurch gekennzeichnet, dass** die Verbindung in der Zusammensetzung in einer Konzentration zwischen 0,1% und 5% (Gew./Gew.) eingeschlossen vorliegt.

6. Zusammensetzung nach Anspruch 5, **dadurch gekennzeichnet, dass** die Verbindung in der Zusammensetzung in einer Konzentration zwischen 0,2% und 1% (Gew./Gew.) eingeschlossen vorliegt.

7. Zusammensetzung nach einem der Ansprüche 1 bis 6, **dadurch gekennzeichnet, dass** sie außerdem wenigstens ein anderes Lichtschutzmittel enthält.

8. Zusammensetzung nach einem der Ansprüche 1 bis 7, **dadurch gekennzeichnet, dass** sie wenigstens eine Verbindung enthält, welche in der Gruppe, die aus den das Sonnenlicht abschirmenden Verbindungen, den das Sonnenlicht filternden Verbindungen und den Fängern von freien Radikalen besteht, ausgewählt wird.

9. Zusammensetzung nach einem der Ansprüche 1 bis 8, **dadurch gekennzeichnet, dass** sie außerdem das Protein HSP 32 oder eines seiner aktiven Fragmente enthält.

10. Verfahren zur kosmetischen Behandlung der Haut oder der oberflächlichen Körperauswüchse oder -wucherungen in Hinblick darauf, diese vor den schädlichen Wirkungen von Strahlungen, insbesondere der ultravioletten Strahlungen zu schützen, **dadurch gekennzeichnet, dass** man vor oder zum Zeitpunkt einer Exposition gegenüber diesen Strahlungen örtlich eine wirksame Menge von wenigstens einer Zusammensetzung nach einem der Ansprüche 1 bis 9 aufträgt.

11. Verfahren nach Anspruch 10, **dadurch gekennzeichnet, dass** es dazu bestimmt ist, die Bildung von Sonnenerythemen, von Sonnenallergien und von Sonnenelastose zu bekämpfen.

12. Verfahren nach Anspruch 10, **dadurch gekennzeichnet, dass** es dazu bestimmt ist, die aktinische Alterung der Haut zu verhindern oder zu verzögern, insbesondere das Auftreten von Falten aufgrund der schädlichen Wirkungen der ultravioletten Strahlen zu verhindern oder zu verzögern.

## Claims

1. Dermatological or cosmetological composition for external topical administration, **characterized in that** it comprises, together with pharmaceutically and/or cosmetically acceptable excipients:
- at least one anti-ultraviolet A and/or anti-ultraviolet B screening agent,
- at least one compound capable of activating the endogenous synthesis of HSP 32 or a functional peptide fragment of such a protein, and
- forskolin or any extract containing it; in particular, extracts of Plectranthus barbatus.

2. Composition according to Claim 1, **characterized in that** the compound capable of activating the endogenous synthesis of HSPs is selected from PCOs and derivatives thereof, caffeic acid esters and derivatives thereof.

3. Composition according to Claim 2, **characterized in that** the PCO derivative is a crosslinked PCO.

4. Composition according to Claim 2, **characterized in that** the PCO is a PCO from grape seed or a PCO from green tea.

5. Composition according to any one of Claims 1 to 4, **characterized in that** said compound is present in a concentration of between 0.1% and 5% w/w in the composition.

6. Composition according to Claim 5, **characterized in that** said compound is present in a concentration of between 0.2% and 1% w/w in the composition.

7. Composition according to any one of Claims 1 to 6, **characterized in that** it also contains at least one other photoprotective agent.

8. Composition according to any one of Claims 1 to 7, **characterized in that** it contains at least one compound selected from the group consisting of physical sunblocks, sunscreens and free-radical scavengers.

9. Composition according to one of Claims 1 to 8, **characterized in that** it also contains the protein HSP 32 or an active fragment thereof.

10. Cosmetic method for treating the skin or integuments in order to protect them against the harmful effects of radiation, in particular ultraviolet radiation, **characterized in that** an effective amount of at least one composition according to any one of Claims 1 to 9 is applied locally, before or at the time of exposure to said radiation.

11. Method according to Claim 10, **characterized in that** it is intended to combat the formation of solar erythema, solar allergies or solar elastosis.

12. Method according to Claim 10, **characterized in that** it is intended to prevent or delay actinic ageing of the skin, in particular to prevent or delay the appearance of wrinkles caused by the harmful effects of ultraviolet radiation.
